(19) Europäisches Patentamt
European Patent Office
Office européen des brevets

(11) Numéro de publication : **0 528 729 A1**

(12) # DEMANDE DE BREVET EUROPEEN

(21) Numéro de dépôt : **92402293.2**

(22) Date de dépôt : **17.08.92**

(51) Int. Cl.[5] : **C07C 269/06,** C07C 231/12, C07D 305/14, C07C 233/83, C07C 271/22

(30) Priorité : **19.08.91 FR 9110398**

(43) Date de publication de la demande : **24.02.93 Bulletin 93/08**

(84) Etats contractants désignés : **PT**

(71) Demandeur : **RHONE-POULENC RORER SA
20, avenue Raymond Aron
F-92160 Antony (FR)**

(72) Inventeur : **Denis, Jean-Noel
Gites de Beledonne, Apt. No. 3, Le Pinet
F-38410 Uriage (FR)**
Inventeur : **Greene, Andrew
La Maison du Verger, Saint Martin d'Uriage
F-38410 Uriage (FR)**
Inventeur : **Kanazawa, Alice
97 cours Berria
F-38000 Grenoble (FR)**

(74) Mandataire : **Pilard, Jacques et al
RHONE-POULENC RORER S.A. Direction
Brevets 20 avenue Raymond Aron
F-92165 Antony Cédex (FR)**

(54) **Procédé de préparation de dérivés de la beta-phenylisoserine et leur utilisation.**

(57) Procédé de préparation de nouveaux dérivés de la β-phénylisosérine de formule générale (I) et leur emploi pour préparer des dérivés du taxane biologiquement actifs.

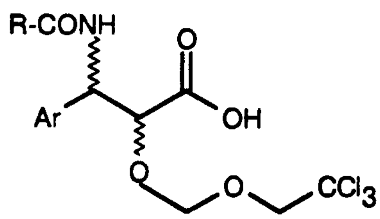

(I)

EP 0 528 729 A1

La présente invention concerne un procédé de préparation de nouveaux dérivés de la β-phénylisosérine de formule générale:

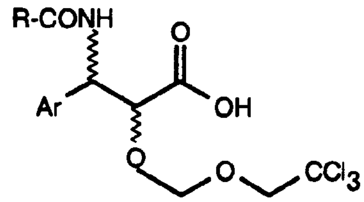

(I)

dans laquelle R représente un radical t-butoxy ou phényle et Ar représente un radical aryle choisi parmi les radicaux phényle ou α- ou β-naphtyle éventuellement substitués par un ou plusieurs atomes ou radicaux, identiques ou différents, choisis parmi les atomes d'halogène et les radicaux alcoyles, alkyles, alcynyles, alcoyloxy, alcoylthio, hydroxy, mercapto, amino, monoalcoylamino, dialcoylamino, acylamino, alcoxycarbonylamino, carboxy, carbamoyle, N,N-dialcoylcarbamoyle, cyano, nitro ou trifluorométhyle, sous forme racémique ou énantiomériquement pure ou sous forme de leurs mélanges éventuellement sous forme de sel ou d'ester.

Les produits de formule générale (I) sont particulièrement utiles pour préparer les dérivés du taxane de formule générale:

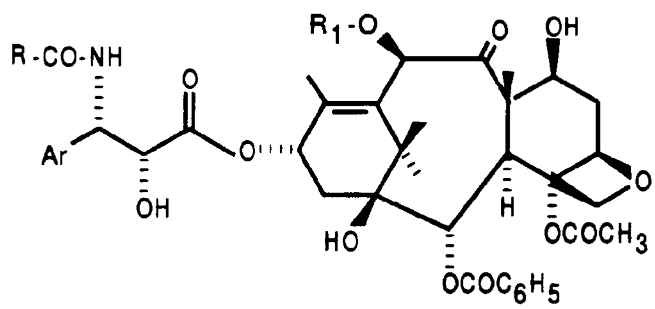

(II)

dans laquelle R et Ar sont définis comme précédemment et $R_1$ représente un atome d'hydrogène ou un radical acétyle, qui présentent des propriétés antileucémiques et antitumorales remarquables.

Selon les brevets américains US 4 924 011 et US 4 924 012, il est connu de préparer les dérivés du taxane de formule (II), dans laquelle Ar représente un radical phényle par condensation d'un dérivé de la β-phénylisosérine de formule générale:

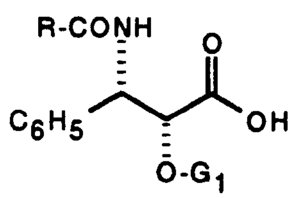

(III)

dans laquelle R est défini comme précédemment et $G_1$ représente un groupement protecteur de la fonction hydroxy, qui est en particulier le radical éthoxy-1 éthyle, sur un dérivé de la baccatine III ou de la désacétyl-10 baccatine III de formule générale:

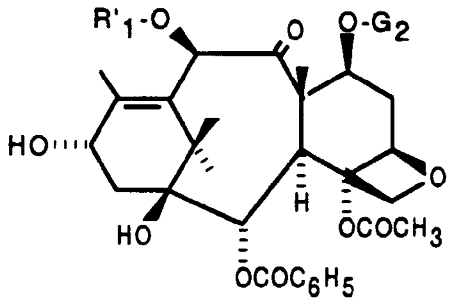

(IV)

dans laquelle $R'_1$ représente un radical acétyle ou un groupement protecteur de la fonction hydroxy et $G_2$ représente un groupement protecteur de la fonction hydroxy, suivie du remplacement des groupements protecteurs, qui sont choisis de préférence parmi les radicaux trichloro-2,2,2 éthoxycarbonyle et trialcoylsilyle dont chaque partie alcoyle contient 1 à 4 atomes de carbone, par des atomes d'hydrogène.

Cependant, le produit de formule générale (III), en particulier lorsque $G_1$ représente un radical éthoxy-1 éthyle, n'est pas très stable ce qui nécessite des précautions particulières pour sa synthèse et son stockage.

Il doit être considéré que la mise en oeuvre d'un produit de formule générale (III) non totalement protégé peut entraîner, lors de sa condensation avec le dérivé de la désacétyl-10 baccatine III, de formule générale (IV), des baisses considérables de rendement.

Par ailleurs, l'utilisation comme groupement protecteur du radical éthoxy-1 éthyle introduit, d'une part, un radical méthyle qui pot avoir une influence stérique sur l'estérification et, d'autre part, un atome de carbone asymétrique non résolu ce qui entraîne la présence de deux stéréoisomères rendant difficiles les processus de purification.

Il a maintenant été trouvé que le groupement protecteur trichloro-2,2,2 éthoxyméthyle du produit de formule générale (I) présente les avantages suivants:

- il est très stable dans les conditions usuelles de basicité et d'acidité,
- il ne possède ni atome de carbone asymétrique entraînant des problèmes de stéréoisométrie ni de groupement induisant des effets stériques,
- il est facilement introduit dans des conditions douces et non épimérisantes,
- il est facilement éliminé dans les conditions qui permettent le remplacement des groupements protecteurs trichloro-2,2,2 éthoxycarbonyle du cycle taxane par des atomes d'hydrogène.

La présente invention a donc pour objet la préparation du nouveau produit de formule générale (I) éventuellement sous forme de sel ou d'ester et son utilisation pour préparer les dérivés du taxane de formule générale (II).

Selon l'invention, le produit de formule générale (I) peut être obtenu par action d'un halogénure, de préférence le bromure, de trichloro-2,2,2 éthoxyméthyle sur un produit de formule générale qui peut être sous forme racémique ou énantiomériquement pure ou sous forme de leurs mélanges:

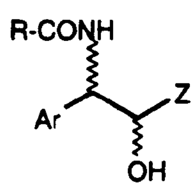

(V)

dans laquelle Z représente une fonction ester ($-COOR_2$) ou une double liaison vinylique ($-CH=CH_2$), suivi selon le cas de la saponification ou de l'oxydation du produit obtenu.

Généralement, l'action de l'halogénure de trichloro-2,2,2 éthoxyméthyle sur le produit de formule générale (V) est réalisée en opérant dans un solvant organique anhydre tel que l'acétonitrile à une température comprise entre 0 et 50°C, et, de préférence, voisine de 20°C. Il est particulièrement avantageux d'opérer en présence d'un accepteur de protons tel que le 1,8-bis-(diméthylamino) naphtalène (ou "éponge à protons"), de méthyl-2 butène-2 et de déshydratant tel que le tamis moléculaire 3 Å ou 4 Å.

Lorsque Z représente une fonction ester-$COOR_2$, $R_2$ étant un radical alcoyle contenant 1 à 4 atomes de carbone éventuellement substitué par un radical phényle, le produit obtenu est saponifié en acide de formule générale (I).

Généralement la saponification est effectuée au moyen d'une base minérale dans des conditions qui ne touchent pas au reste de la molécule. La saponification peut être effectuée au moyen d'un carbonate alcalin

tel que le carbonate de potassium en milieu hydroalcoolique tel qu'un mélange eau-méthanol.

Lorsque Z représente une liaison vinylique $-CH=CH_2$, le produit obtenu est oxydé en acide de formule générale (I).

Généralement l'oxydation est réalisée au moyen d'un periodate tel que le periodate de sodium en présence d'une quantité catalytique d'un sel de ruthénium tel que le chlorure de ruthénium $RuCl_3$. La réaction est mise en oeuvre en milieu hydroorganique homogène ou hétérogène. Comme solvant peuvent être utilisé l'acétonitrile et le tétrachlorure de carbone. La réaction peut être effectuée en milieu basique par exemple en présence d'hydrogénocarbonate de sodium.

L'oxydation peut être aussi réalisé au moyen de de permanganate de potassium, par exemple en présence d'adogen dans un mélange pentane-eau ou en présence d'aliquat ou de dicyclohexyl- 18 crown-6 dans le dichlorométhane ou dans un mélange pyridine-eau.

Peut aussi être utilisé le permanganate de triéthylbenzylammonium en présence de pyridine dans le dichlorométhane.

Le produit de formule générale (V) dans laquelle Z représente une fonction ester ($-COOR_2$) peut être préparé selon les méthodes connues.

Le produit de formule générale (V) dans laquelle Z représente une double liaison vinylique ($-CH=CH_2$)) peut être obtenu:

- par action de l'acroléine sur un dérivé de la benzylamine de formule générale:

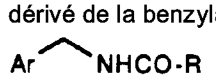 (VI)

dans laquelle R et Ar sont définis comme précédemment, après double anionisation.

La double anionisation du produit de formule générale (VI) s'effectue généralement en utilisant 2 équivalents d'un dérivé organolithié tel que le s-butyllithium, en opérant dans un solvant organique anhydre tel que le tétarhydrofuranne à une température inférieure à -50°C.

La réaction de l'acroléine avec le dianion du produit de formule (VI) est généralement effectuée en ajoutant l'acroléine, de préférence fraîchement distillée à la solution du dianion préalablement refroidie à une température comprise entre -50 à -100°C, de préférence voisine de -100°C. Après hydrolyse, on obtient le produit de formule (V) sous forme d'un mélange des diastéréoisomères syn et anti dont on sépare éventuellement la forme syn par chromatographie,

- par action d'un halogénure de vinylmagnésium sur l'aldéhyde obtenu par oxydation d'un alcool de formule générale :

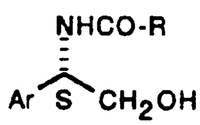 (VII)

dans laquelle R et Ar sont définis comme précédemment, qui est lui-même obtenu en traitant par un agent réducteur et par un réactif permettant d'introduire un groupement t-butoxycarbonyle ou benzoyle, un aminoacide de formule générale:

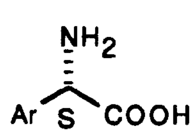 (VIII)

dans laquelle Ar est défini comme précédemment.

Généralement l'alcool de formule générale (V) est obtenu en ajoutant l'aldéhyde à une solution d'un halogénure de vinylmagnésium, de préférence le bromure de vinylmagnésium, dans un solvant organique inerte tel que le tétrahydrofuranne éventuellement en mélange avec du chlorure de méthylène.

Généralement, l'oxydation de l'alcool de formule (VII) est réalisée au moyen du mélange chlorure d'oxalyle-diméthylsulfoxyde à une température inférieure à 0°C dans un solvant organique tel que le chlorure de méthylène ou le tétrahydrofuranne en présence d'une base organique telle que la triéthylamine ou la diisopropyléthylamine.

Il n'est pas nécessaire d'isoler l'aldéhyde pour faire réagir l'halogénure de vinylmagnésium.

L'alcool de formule (VII) peut être obtenu :

- soit par action d'un agent réducteur permettant d'introduire un groupement t.butoxycarbonyle ou benzoyle sur l'aminoalcool obtenu par réduction de l'aminoacide de formule générale (VIII),
- soit par action d'un agent réducteur sur l'acide obtenu par action d'un agent permettant d'introduire un groupement t-butoxycarbonyle ou benzoyle sur l'aminoacide de formule générale (VIII).

Comme agent réducteur, on utilise de préférence l'hydrure double de lithium et d'aluminium ou le borane ($BH_3$), de préférence sous forme de complexe avec le diméthylsulfure en présence d'éthérate de trifluorure de bore. Généralement la réaction s'effectue dans un solvant organique tel que par exemple un éther comme le tétrahydrofuranne ou le diméthoxyéthane. Généralement la réduction s'effectue à une température comprise entre 50 et 100°C.

Comme agent permettant d'introduire un groupement t-butoxycarbonyle ou benzoyle, on utilise de préférence, selon le cas, le dicarbonate de di-t-butyle ou le chlorure de benzoyle. Généralement on opère dans un solvant organique tel que le chlorure de méthylène ou un éther comme le tétrahydrofuranne ou le diméthoxyéthane en présence d'une base minérale telle que la soude ou le bicarbonate ou carbonate de sodium ou d'une base organique telle que la triétylamine ou la diméthylamino-4 pyridine. Généralement la réaction s'effectue entre 0°C et la température de reflux du mélange réactionnel.

L'acide de formule générale (I), éventuellement sous forme de sel ou d'ester, obtenu selon le procédé de la présente invention peut être purifié par des méthodes physiques telles que la cristallisation ou la chromatographie. Le produit de formule générale (I) (forme syn ou syn-anti, mélange racémique) peut également être dédoublé en ses énantiomères syn, par exemple par formation d'un sel avec une base optiquement active telle que l'éphédrine ou la pseudo-éphédrine puis hydrolyse du sel obtenu.

L'acide de formule générale (I) est utile, en particulier, pour préparer les dérivés du taxane de formule générale (II).

Les produits de formule générale (II) sont obtenus par estérification de l'alcool de formule générale (IV), suivi du remplacement des groupements protecteurs des fonctions hydroxy par des atomes d'hydrogène et éventuellement de la séparation des énantiomères 2R,3S.

Généralement l'estérification est effectuée dans un hydrocarbure aromatique (benzène, toluène, xylène) à une température comprise entre 20 et 80°C en présence d'un agent de condensation tel que le dicyclohexylcarbodiimide ou le 2-dipyridylcarbonate et d'un agent d'activation tel que la diméthylamino-4 pyridine. Généralement on utilise un excès d'acide de formule générale (I) par rapport à l'alcool de formule générale (IV).

Le remplacement par des atomes d'hydrogène des groupements protecteurs $R'_1$, $G_1$ et $G_2$, lorsqu'ils représentent un radical trichloro-2,2,2 éthoxycarbonyle ou trichloro-2,2,2 éthoxyméthyle s'effectue généralement au moyen de zinc ou d'un couple zinc-cuivre en présence d'acide acétique et éventuellement d'un alcool contenant 1 à 3 atomes de carbone à une température voisine de 60°C. Dans le cas où $G_2$ est un radical trialcoylsilyle dont chaque partie contient 1 à 4 atomes de carbone, le remplacement de ce radical par un atome d'hydrogène d'effectue par un traitement ultérieur acide (par exemple HCl hydroalcoolique).

Les exemples suivants illustrent la présente invention.

EXEMPLE 1

Dans un ballon de 10 cm3, muni d'une agitation magnétique, on introduit sous atmosphère d'argon, 100 mg (0,339 mmole) de t-butoxycarbonylamino-3 hydroxy-2 phényl-3 propionate de méthyle-(2R,3S) et 1,2 cm3 d'acétonitrile anhydre. On ajoute ensuite succesivement 214,3 mg (1 mmole) d'"éponge à protons", 718 µl (475,5 mg; 6,78 mmoles) de méthyl-2 butène-2 et 5 grains de tamis moléculaire 4Å. On agite pendant 10 minutes à une température voisine de 20°C puis on ajoute 164,3 mg (0,678 mmole) de bromure de trichloro-2,2,2 éthoxyméthyle. On laisse réagir pendant 24 heures à une température voisine de 20°C. Le mélange devient hétérogène au fur et à mesure de l'avancement de la réaction. On ajoute encore successivement 214,3 mg (1 mmole) d'"éponge à protons" et 164,3 mg (0,678 mmole) de bromure de trichloro-2,2,2 éthoxyméthyle. On laisse réagir pendant 24 heures à une température voisine de 20°C. On ajoute alors 3 cm3 d'une solution aqueuse saturée de bicarbonate de sodium. Après 10 minutes d'agitation, on ajoute ensuite 40 cm3 de chlorure de méthylène et 5 cm3 d'eau. Apès décantation, la phase aqueuse est lavée 2 fois avec 10 cm3 de chlorure de méthylène. Les phases organiques réunies sont lavées avec 2 fois 5 cm3 d'une solution aqueuse d'acide chlorhydrique 2M, 4 fois avec 5 cm3 d'eau et 2 fois avec 5 cm3 d'une solution aqueuse saturée de chlorure de sodium. Après séchage sur sulfate de sodium anhydre, filtration et concentration à sec sous pression réduite, on obtient 290 mg de produit qui est purifié par chromatographie sur silice en éluant avec un mélange hexane-éther (8-2 en volumes). On obtient ainsi, avec un rendement de 86 %, 133 mg (0,291 mmole) de t-butoxycarbonylamino-3 phényl-3 (trichloro-2,2,2 éthoxyméthoxy)-2 propionate de méthyle-(2R,3S) dont les caractéristiques sont les suivantes:

- point de fusion : 90-91°C (cyclohexane)

- pouvoir rotatoire: $[\alpha]^{25}_D$ = +41,8° (c = 1,33; chloroforme)
- spectre infra-rouge (film) : principales bandes d'absorption caractéristiques à 3450, 3000, 2925, 1760, 1720, 1500, 1460, 1440, 1370, 1175, 1130, 1090, 1020, 950 et 810 $cm^{-1}$
- spectre de résonance magnétique nucléaire du proton (chloroforme deutéré ; 200 MHz) : 1,41 (s, 9H) ; 3,42 ($AB_q$, $J_{AB}$ = 11,5 Hz, $\delta_A$-$\delta_B$ = 91,3 Hz, 2H) ; 3,80 (s, 3H); 4,54 (s déformé, 1H); 4,79 ($AB_q$, $J_{AB}$ = 7,3 Hz, $\delta_A$-$\delta_B$=26 Hz, 2H); 5,36 (s large, 1H) ; 5,46 (s large, 1H) ; 7,23-7,41 (m, 5H).
- spectre de résonance magnétique nucléaire du $^{13}C$ (chloroforme deutéré, 75,47 MHz) : 28,25 ($CH_3$) ; 52,47 ($OCH_3$) ; 55,89 (CH) ; 77,44 (CH) ; 79,40 ($CH_2$) ; 80,02 (C) ; 94,75 ($CH_2$) ; 96,21 (C) ; 126,43 (CH) ; 127,82 (CH) ; 128,63 (CH) ; 139,01 (C) ; 155,04 (C) ; 169,98 (C)
- spectre de masse (i.c. ; $NH_3$ + isobutane: ion moléculaire (massif) : $M^+$ (456,5) ; $M^+$ + 2 (458,5) ; $M^+$ + 4 (460,5) ; $M^+$ + 6 (462,5) : 4 pics dans le rapport 100-97,5-31,7 et 3,4
- analyse élémentaire ($C_{18}H_{24}O_6NCl_3$)

| | | | | | | |
|---|---|---|---|---|---|---|
| calculé | C % | 47,33 | H % | 5,30 | N % | 3,07 |
| trouvé | | 47,45 | | 5,15 | | 3,19 |

EXEMPLE 2

Dans un ballon de 100 cm3, muni d'une agitation magnétique, on introduit 196 mg (0,43 mmole) de l'ester obtenu à l'exemple 1 et 24 cm3 de méthanol. On ajoute ensuite à la solution obtenue, 12 cm3 d'eau distillée et 178 mg (1,29 mmole) de carbonate de potassium. On agite pendant 24 heures une température voisine de 20°C puis évapore sous pression réduite le méthanol et l'eau. Le résidu obtenu est dissous dans le minimum d'eau (12 cm3) en chauffant légèrement. La phase aqueuse basique est lavée 3 fois avec 10 cm3 d'éther. La phase aqueuse est refroidie à 0°C puis est acidifiée par 4 cm3 d'acide chlorhydrique 2M, sous forte agitation, en présence de 30 cm3 de chlorure de méthylène. Après décantation, elle est ensuite extraite 8 fois avec 20-25 cm3 de chlorure de méthylène. Les phases organiques réunies sont lavées 4 fois avec 10 cm3 d'eau puis avec 10 cm3 d'une solution aqueuse saturée de chlorure de sodium. Après séchage sur sulfate de magnésium anhydre, filtration et concentration à sec sous pression réduite, on obtient, avec un rendement de 89 %, 170 mg (0,384 mmole) d'acide t-butoxycarbonylamino-3 phényl-3 (trichloro-2,2,2 éthoxyméthoxy)-2 propionique-(2R,3S) pur sous forme d'un solide blanc dont les caractéristiques sont les suivantes :

- pouvoir rotatoire:$[\alpha]^{20}_D$ = +62,2° (c = 1, chloroforme)
- spectre infra-rouge (film) : principales bandes d'absorption caractéristiques à 3500-2300, 3000, 2940, 1760, 1730, 1500, 1460, 1400, 1375, 1260, 1180, 1135, 1090, 1020 et 810 $cm^{-1}$
- spectre de résonance magnétique nucléaire du proton (chloroforme deutéré ; 200 MHz) : 1,43 (s, 9H) ; 2,73 (s large, 1H + $H_2O$) ; 3,44 ($AB_q$, $J_{AB}$ = 11,7 Hz, $\delta_A$-$\delta_B$ = 91 Hz, 2H) ; 4,60 (s, 1H) ; 4,83 ($AB_q$, $J_{AB}$ = 7,3 Hz, $\delta_A$-$\delta_B$ = 30 Hz, 2H) ; 5,43 (d déformé, J = 9,5 Hz, 1H) ; 5,59 (d déformé, J = 9 Hz, 1H) ; 7,29-7,38 (m, 5H)
- spectre de résonance magnétique nucléaire du $^{13}C$ (chloroforme deutéré ; 75,47 MHz) : 28,25 ($CH_3$) ; 55,78 (CH) ; 57,88 (CH) ; 79,43 ($CH_2$) ; 80,84 (C) ; 94,81 ($CH_2$) ; 96,22 (C) ; 126,39 (CH) ; 127,89 (CH) ; 128,66 (CH) ; 138,92 (C) ;155,69 (C) et 171,91 (C)
- spectre de masse (i.c. ; $NH_3$ + isobutane) : ion moléculaire (massif) : $M^+$ (442,5) ; 31,7 et 3,4
- analyse élémentaire: ($C_{17}H_{22}O_6NCl_3$, 1$H_2O$)

| | | | | | | |
|---|---|---|---|---|---|---|
| calculé | C % | 44,32 | H % | 5,25 | N % | 3,04 |
| trouvé | | 44,49 | | 5,26 | | 3,06 |

EXEMPLE 3

Dans un ballon de 15 cm3, muni d'une agitation magnétique, on introduit, sous atmosphère d'argon, 263 mg (1 mmole) de (±) phényl-1 t-butoxycarbonylamino-1 hydroxy-2 butène-3 syn et 3,6 cm3 d'acétonitrile anhydre. On ajoute ensuite, successivement, à la solution résultante, 2,12 cm3 (1,4 g ; 20 mmoles) de méthyl-2 butène-2, 10 grains de tamis moléculaire 4 Å et 643 mg (3 mmoles) d'"éponge à protons". On agite pendant 5 minutes à une température voisine de 20°C puis on ajoute 485 mg (2 mmoles) de bromure de trichloro-2,2,2 éthoxyméthyle. On agite pendant 24 heures à une température voisine de 20°C puis on ajoute 643 mg (3 mmoles) d'"éponge à protons" et 485 mg (2 mmoles) de bromure de trichloro-2,2,2 éthoxyméthyle. Après 24 heures de réaction on ajoute à nouveau 242,4 mg (1 mmole) de bromure de trichloro-2,2,2 éthoxyméthyle puis agite

encore pendant 24 heures à une température voisine de 20°C. On ajoute alors une solution aqueuse saturée de bicarbonate de sodium. On agite pendant 10 minutes. On ajoute alors 100 cm3 de chlorure de méthylène et 5 cm3 d'eau. Après décantation, la phase aqueuse est extraite 2 fois avec 20 cm3 de chlorure de méthylène. Les phases organiques réunies sont lavées 2 fois avec 10 cm3 d'une solution aqueuse d'acide chlorhydrique 2M, 4 fois avec 10 cm3 d'eau, 2 fois avec 10 cm3 d'une solution aqueuse saturée de sodium, puis séchées sur sulfate de sodium anhydre. Après filtration et concentration à sec sous pression réduite, on obtient 868 mg d'un produit qui est purifié par chromatographie sur silice en éluant avec un mélange hexane-éther (9-1 en volumes). On obtient ainsi, avec un rendement de 70 %, 298 mg (0,70 mmole) de (±) phényl-1 t-butoxycarbonylamino-1 (trichloro-2,2,2 éthoxyméthoxy)-2-butène-3 syn dont les caractéristiques sont les suivantes:

- point de fusion: 75°C (hexane)
- spectre infra-rouge (film) : bandes d'absorption caractéristiques à 3460, 3380, 3080, 3050, 3020, 3000, 2950, 2910, 1720, 1500, 1400, 1370, 1290, 1255, 1170, 1130, 1080, 1020, 940 et 810 $cm^{-1}$
- spectre de résonance magnétique nucléaire du proton (chloroforme deutéré; 200 MHz) : 1,43 (s, 9H) ; 3,42 ($AB_q$, $J_{AB}$ = 11,8 Hz, $\delta_A$-$\delta_B$ = 74 Hz, 2H) ; 4,37 (dd déformé, J = 2,5 Hz et 7 Hz, 1H) ; 4,74 ($AB_q$, $J_{AB}$ = 7 Hz, $\delta_A$-$\delta_B$ = 17,7 Hz, 2H) ; 4,86 (s large, 1H) ; 5,35 (d, J = 11 Hz, 1H) ; 5,36 (d, J = 17 Hz, 1H) ; 5,32-5,41 (m, 1H) ; 5,83 (ddd, J = 7 Hz, 11 Hz et 17 Hz, 1H) ; 7,26-7,40 (m, 5H)
- spectre de résonance magnétique nucléaire du $^{13}C$ (chloroforme deutéré ; 75,47 MHz) : 28,34 ($CH_3$) ; 57,64 (CH) ; 79,16 ($CH_2$) ; 79,68 (C) ; 79,98 (CH) ; 92,76 ($CH_2$) ; 96,60 (C) ; 119,67 ($CH_2$) ; 126,65 (CH) ; 127,43 (CH) ; 128,40 (CH); 134,25 (CH) ; 140,31 (C) et 155,48 (C)
- spectre de masse (i.c. ; $NH_3$ + isobutane) : ion moléculaire (massif) : $M^+$ (426,5) ; $M^+$ + 2 (426,5) ; $M^+$ + 4 (428,5) ; $M^+$ + 6 (430,5) : 4 pics avec rapport 100 ; 97,5 ; 31,7 et 3,4
- analyse élémentaire: ($C_{18}H_{24}O_4NCl_3$)

| | | | | | | |
|---|---|---|---|---|---|---|
| calculé | C % | 50,90 | H % | 5,69 | N % | 3,30 |
| trouvé | | 50,81 | | 5,80 | | 3,29 |

Le (±)-phényl-1 t-butylcarbonylamino-1 hydroxy-2 butène-3 syn est obtenu pur à partir du benzylcarbamate de t-butyle selon le mode opératoire décrit dans la demande de brevet français FR 2 662 440.

Dans un ballon de 10 cm3, muni d'une agitation magnétique, on introduit, sous atmosphère d'argon, 245 mg (0,577 mmole) du produit obtenu précédemment en solution dans 1,2 cm3 d'acétonitrile. On ajoute sucessivement 1,2 cm3 de tétrachlorure de carbone, 1,8 cm3 d'eau distillée et, sous forte agitation, 315 mg (3,75 mmoles) de bicarbonate de sodium solide. On ajoute ensuite, par petites fractions, 680 mg (3,18 mmoles) de periodate de sodium. On laisse réagir le mélange réactionnel hétérogène jusqu'à cessation du dégagement gazeux puis on ajoute 24,5 mg de $RuCl_3$. On laisse réagir sous forte agitation pendant 29 heures une température voisine de 20°C puis on dilue le mélange fractionnel avec de l'eau jusqu'à l'obtention d'un volume total de 20 cm3. La phase aqueuse basique est lavée 3 fois avec 10 cm3 d'éther puis est refroidie à 0°C. En présence de 40 cm3 de chlorure de méthylène, on ajoute, sous forte agitation, 2 cm3 d'acide chlorhydrique 2M. La phase aqueuse est extraite 8 fois avec 40 cm3 de chlorure de méthylène. Les phases organiques sont réunies et lavées 3 fois avec 10 cm3 d'eau puis avec 10 cm3 d'une solution aqueuse saturée de chlorure de sodium et enfin séchées sur un mélange sulfate de sodium sulfate de magnésium anhydre. Après filtration sous pression réduite sur célite et concentration à sec, on obtient, avec un rendement de 80 %, 204 mg (0,461 mmole) d'acide (±) t-butoxycarbonylamino-3 phényl-3 (trichloro-2,2,2 éthoxyméthoxy)-2 propionique.

## EXEMPLE 4

Dans un ballon de 10 cm3, on introduit 215 mg (0,48 mmole) d'acide (±)-t-butoxycarbonylamino-3 phényl-3 (trichloro-2,2,2 éthoxyméthoxy)-2 propionique (syn) (obtenu dans les conditions de l'exemple 3) et 1,2 cm3 d'acétone. On chauffe légèrement la solution obtenue jusqu'à l'obtention d'une solution homogène parfaite puis on ajoute une solution de 85 mg (0,51 mmole) de (+)-éphédrine dans 1,2 cm3 d'acétone. On agite le mélange réactionnel jusqu'à l'obtention d'une solution homogène puis on laisse cristalliser en refroidissant à une température voisine de 20°C. Les cristaux blancs obtenus sont séparés par filtration et lavés 2 fois avec de l'éther. On obtient ainsi 130 mg de sel d'éphédrine de l'acide t.butoxycarbonylamino-3 phényl-3 (trichloro-2,2,2 éthoxyméthoxy)-2 propionique-(2R,3S).

Les cristaux ainsi obtenus sont dissous à chaud dans 2 cm3 de méthanol. On ajoute 0,5 cm3 d'acétate d'éthyle puis on refroidit à une température voisine de 20°C. Les cristaux blancs formés sont séparés par filtration puis traités par l'acide chlorhydrique 1N aqueux dans le dichlorométhane à une température voisine de 20°C. Après extraction selon les méthodes habituelles, on obtient, avec un rendement de 30,1 %, 64 mg

(0,1446 mole) d'acide (+)-t.butoxycarbonylamino-3 phényl-3 (trichloro-2,2,2 éthoxyméthoxy)-2 propionique-(2R,3S) pour lequel l'excès énantiomérique est supérieur à 99 % (détermination à partir des esters de Mosher des alcools-esters racémique et optiquement pur correspondants) et dont les spectres infra-rouge et de résonance magnétique nucléaire du proton sont identiques à ceux du produit de l'exemple 2.

Les eaux-mères obtenues lors de la première cristallisation sont concentrées à sec. Le résidu est dissous à chaud dans 2,5 cm3 de méthanol. On ajoute 0,5 cm3 d'acétate d'éthyle. Après refroidissement à 20°C, on obtient, après séparation par filtration, 78 mg de cristaux blancs que l'on mélange au résidu obtenu par concentration à sec des eaux-mères de cristallisation de l'acide (+)-t.butoxycarbonylamino-3 phényl-3 (trichloro-2,2,2 éthoxyméthoxy)-2 propionique-(2R,3S). Après recristallisation du mélange dans le mélange méthanol-acétate d'éthyle, on obtient 33,5 mg de cristaux blancs que l'on traite par l'acide chlorhydrique aqueux 1N dans le dichlorométhane à 20°C. Après extraction dans les conditions habituelles, on obtient 24,5 mg (0,055 mmole) d'acide (+)-t.butoxycarbonylamino-3 phényl-3 (trichloro-2,2,2 éthoxyméthoxy)-2 propionique-(2R,3S) pour lequel l'excès énantiomérique est supérieur à 99 %.

Au total, on obtient 88,5 mg d'acide (+)-t.butoxycarbonylamino-3 phényl-3 (trichloro-2,2,2 éthoxyméthoxy)-2 propionique-(2R,3S) dont le pouvoir rotatoire est: $[\alpha]^{20}_D$ = +60° (c = 0,92 ; chloroforme).

Le rendement de la résolution est de 83 %.

## EXEMPLE 5

Dans un ballon de 15 cm3, muni d'une agitation magnétique, on introduit, sous atmosphère d'argon, 202 mg (0,77 mmole) de (±)-t.butoxycarbonylamino-1 phényl-1 hydroxy-2 butène-3 pour lequel le rapport syn/anti est égal à 6/1 et 2,8 cm3 d'acétonitrile anhydre. A la solution obtenue, on ajoute successivement 1,63 cm3 (1,08 g ; 15,4 mmoles) de méthyl-2 butène-2, 8 grains de tamis moléculaire 4Å et 494 mg (2,3 mmoles) d'"éponge à protons". On agite pendant 5 minutes à une température voisine de 20°C, puis on ajoute 381 mg (1,57 mmole) de bromure de trichloro-2,2,2 éthoxyméthyle. On agite pendant 24 heures à une température voisine de 20°C puis on ajoute 494 mg (2,3 mmoles) d'"éponge à protons" et 381 mg (1,57 mmole) de bromure de trichloro-2,2,2 éthoxyméthyle. Après 24 heures d'agitation, on ajoute à nouveau 190 mg (0,785 mmole) de bromure de trichloro-2,2,2 éthoxyméthyle puis on agite pendant 24 heures à 20°C puis pendant 20 heures à 25-30°C.

On traite le mélange réactionnel par une solution saturée d'hydrogénocarbonate de sodium pendant 10 minutes. On ajoute alors au mélange résultant 80 cm3 de dichlorométhane et 4 cm3 d'eau. Après décantation, la phase aqueuse est extraite 2 fois par 15 cm3 de dichlorométhane. Les phases organiques réunies sont lavées 2 fois par 8 cm3 d'une solution aqueuse d'acide chlorhydrique 2M, 4 fois par 8 cm3 d'eau et 2 fois par 8 cm3 d'une solution saturée de chlorure de sodium. Après séchage sur sulfate de sodium anhydre, filtration et concentration à sec, on obtient 674 mg d'une huile jaune qui est purifiée par chromatographie sur une colonne de gel de silice en éluant avec un mélange hexane-éther (9-1 en volumes).

On obtient ainsi, avec un rendement de 62 %, 202 mg (0,476 mmole) de (±)-t.butoxycarbonylamino-1 phényl-1 (trichloro-2,2,2 éthoxyméthoxy)-2 butène-3 pour lequel le rapport syn/anti est égal à 6/1.

Le (±)-t.butoxycarbonylamino-1 phényl-1 hydroxy-2 butène-3 sous forme du mélange des diastéréoisomères syn et anti dans le rapport 6/1 peut être préparé de la manière suivante:

Dans un ballon de 250 cm3 mis sous argon et muni d'un système d'agitation magnétique, on introduit successivement 4,2 g (20,3 mmoles) de benzylcarbamate de t.butyle, 40 cm3 de tétrahydrofuranne anhydre et 6,5 cm3 (5,01 g, 43,1 mmoles) de tétraméthyléthylènediamine (TMEDA). On refroidit la solution obtenue à -78°C puis on lui additionne, goutte à goutte, 60 cm3 (60 mmoles) d'une solution de butyllithium secondaire 1M dans l'hexane. On laisse réagir pendant 3 heures cette température puis on refroidit à -100°C. On ajoute alors 3 cm3 (2,5 g, 44,9 mmoles) d'acroléine fraîchement distillée, on laisse réagir pendant 3 à 4 minutes à cette température puis pendant 3 heures à -78°C. On hydrolyse le mélange réactionnel résultant à -78°C avec 20 cm3 d'eau, puis on l'extrait avec 2 fois 30 cm3 d'éther. Les phases organiques sont réunies puis elles sont lavées 2 fois avec 20 cm3 d'eau et avec 1 fois 10 cm3 d'une solution aqueuse saturée en chlorure de sodium. Elles sont ensuite séchées sur sulfate de sodium anhydre. Après filtration, les solvants sont éliminés sous pression réduite. Le résidu obtenu (11,6 g) est purifié sur colonne de gel de silice en éluant avec un mélange chlorure de méthylène-éther (95-5 en volumes). On obtient avec un rendement de 49 %, 2,606 g (9,91 mmoles) de t.butoxycarbonylamino-1 phényl-1 hydroxy-2 butène-3, sous forme du mélange des diastéroisomères syn et anti dans le rapport 6/1.

## EXEMPLE 6

Dans un ballon de 10 cm3 muni d'un système d'agitation magnétique, on introduit 102 mg (0,24 mmole)

de (±)-t.butoxycarbonylamino-1 phényl-1 (trichloro-2,2,2 éthoxyméthoxy)-2 butène-3, sous forme du mélange des diastéréoisomères syn et anti dans le rapport 6/1, en solution dans 0,5 cm3 d'acétonitrile. On ajoute ensuite successivement 0,5 cm3 de tétrachlorure de carbone, 0,75 cm3 d'eau distillée et, sous forte agitation, 132 mg (1,57 mmole) d'hydrogénocarbonate de sodium. On ajoute ensuite, par petites portions, 282 mg (1,32 mmole) de periodate de sodium solide. On laisse réagir pendant 5 minutes jusqu'à cessation du dégagement gazeux puis on ajoute, en une seule fois, 10,2 mg de $RuCl_3$. On agite fortement le mélange réactionnel devenu noir pendant 50 heures à une température voisine de 20°C. On ajoute à nouveau 5 mg de $RuCl_3$ puis poursuit l'agitation pendant 40 heures à une température voisine de 20°C. La réaction terminée, on ajoute 6 cm3 d'eau. La phase aqueuse basique est séparée par décantation puis est lavée 3 fois par 4 cm3 d'éther. La phase aqueuse est refroidie à 0°C puis on ajoute sous forte agitation 17 cm3 de dichlorométhane et 0,8 cm3 d'acide chlorhydrique 2M. La phase aqueuse acide est séparée par décantation puis est extraite 8 fois par 17 cm3 de dichlorométhane. Les phases organiques sont réunies puis lavées 4 fois par 4 cm3 d'eau et 2 fois par 4 cm3 d'une solution aqueuse saturée en chlorure de sodium. Après séchage sur sulfate de sodium anhydre, filtration et concentration à sec sous pression réduite, on obtient 75 mg (0,17 mmole) d'acide (±)-t.butoxycarbonylamino-3 phényl-3 (trichloro-2,2,2 éthoxyméthoxy)-2 propionique sous forme du mélange des diastéréoisomères syn et anti dans le rapport 6/1.

Le rendement est de71 %.

EXEMPLE 7

Dans un ballon de 25 cm3, on introduit 701 mg (1,584 mmole) du produit obtenu à l'exemple 6 en solution dans 4 cm3 d'acétone. On chauffe légèrement jusqu'à l'obtention d'une solution homogène. On ajoute alors une solution de 277 mg (1,69 mmole) de (+)-éphédrine dans 4 cm3 d'acétone. On agite jusqu'à l'obtention d'une solution homogène. Après refroidissement à une température voisine de 20°C, les cristaux obtenus sont séparés par filtration et lavés 2 fois par de l'éther. Après séchage, on obtient 385 mg de sel d'éphédrine. Les cristaux ainsi obtenus sont mis en solution à chaud dans un mélange de 6 cm3 de méthanol et de 2 cm3 d'acétate d'éthyle. Après évaporation de 10 % du solvant et refroidissement à une température voisine de 20°C, les cristaux obtenus sont séparés par filtration. On obtient ainsi 190 mg de sel d'éphédrine sous forme de cristaux blancs que l'on traite par l'acide chlorhydrique 1N en présence de dichlorométhane à une température voisine de 20°C. Après extraction selon les méthodes habituelles, on obtient 138 mg (0,313 mmole) d'acide (+)-t.butoxycarbonylamino-3 phényl-3 (trichloro-2,2,2 éthoxyméthoxy)-2 propionique -(2R,3S) pour lequel l'excès énantiomérique est supérieur à 99 %.

Le rendement est de 19,8 %.

Les eaux-mères de recristallisation du sel d'éphérine dans le mélange méthanol-acétate d'éthyle sont concentrées à sec. Le résidu obtenu est dissous à chaud dans un mélange de 3 cm3 de méthanol et de 1 cm3 d'acétate d'éthyle. Après évaporation de 10 % du solvant et refroidissement à une température voisine de 20°C pendant 24 heures, on obtient, après filtration, 92 mg de cristaux blancs que l'on traite par l'acide chlorydrique 1N en présence de dichlorométhane. Après extraction selon les méthodes habituelles, on obtient 65 mg (0,147 mmole) d'acide (+)-t.butoxycarbonylamino-3 phényl-3 (trichloro-2,2,2 éthoxyméthoxy)-2 propionique-(2R,3S) pour lequel l'excès énantiomérique est supérieur à 99 %.

Le rendement est de 9,3 %.

Le traitement des eaux-mères de cristallisation du second jet du sel d'éphédrine dans les conditions décrites ci-dessus (concentration à sec, recristallisation dans un mélange méthanol-acétate d'éthyle, hydrolyse acide) permet d'obtenir 31,4 mg d'acide (+)-t.butoxycarbonylamino-3 phényl-3 (trichloro-2,2,2 éthoxyméthoxy)-2 propionique-(2R,3S) pour lequel l'excès énantiomérique est supérieur à 99 %.

Le rendement est de 4,5 %.

Le rendement global en acide (+)-t.butoxycarbonylamino-3 phényl-3 (trichloro-2,2,2 éthoxyméthoxy)-2 propionique-(2R,3S) est de 33 %. Le rendement de la résolution est voisin de 76 %.

EXEMPLE 8

Dans un ballon de 10 cm3, muni d'une agitation magnétique, on introduit, sous atmosphère d'argon, 119,5 mg (0,27 mmole) de l'acide obtenu à l'exemple 2 en solution dans 2,25 cm3 de toluène anhydre. On ajoute ensuite 55,7 mg (0,27 mmole) de N,N-dicyclohexylcarbodiimide. On laisse pendant 5 minutes puis on ajoute un mélange de 11 mg (0,09 mmole) de N,N-diméthylamino-4 pyridine et de 81 mg (0,09 mmole) d'acétoxy-4 benzoyloxy-2α époxy-5β,20 dihydroxy-1,13α oxo-9 bis-(trichloro-2,2,2 éthoxy) carbonyloxy-7β,10β taxène-11. On laisse réagir pendant 3-4 minutes à une température voisine de 20°C puis on chauffe pendant 24 heures à une température de 72-75°C. Le mélange réactionnel hétérogène est repris par 60 cm3 d'acétate d'éthyle.

La phase organique est lavée avec 5 cm3 d'eau, 2 fois 5 cm3 d'une solution aqueuse saturée de bicarbonate de sodium, 4 fois 5 cm3 d'eau et 2 fois 5 cm3 d'une solution aqueuse saturée de chlorure de sodium, puis enfin séchée sur sulfate de sodium anhydre. Après filtration et concentration à sec sous pression réduite, le résidu obtenu (270 mg) est purifié par chromatographie sur couche mince de silice en éluant avec un mélange chlorure de méthylène-éther (95-5 en volumes; 2 passages).

On obtient ainsi, avec un rendement de 94 %, 112 mg (0,085 mmole) de t-butoxycarbonylamino-3 phényl-3 (trichloro-2,2,2 éthoxyméthoxy)-2 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α dont les caractéristiques sont les suivantes:

- spectre infra-rouge (film): bandes d'absorption caractéristiques à 3450, 3000, 2950, 2910, 1760, 1720, 1500, 1455, 1440, 1380, 1250, 1175, 1090, 1070, 1020, 980, 910, 820, 780 et 720 $cm^{-1}$
- spectre de résonance magnétique nucléaire du proton (chloroforme deutéré ; 300 MHz) : 1,21 (s, 3H) ; 1,30 (s, 3H) ; 1,34 (s, 9H) ; 1,79-1,84 (m, 1H) ; 1,86 (s, 3H) ; 2,04 (s, 3H) ; 2,0-2,08 (m, 1H) ; 2,14-2,46 (m, 2H) ; 2,51 (s, 3H) ; 2,55-2,71 (m, 1H); 3,53 ($AB_q$, $J_{AB}$ = 11,7 Hz, $\delta_A$-$\delta_B$ = 109 Hz, 2H) ; 3,95 (d, J = 7 Hz, 1H) ; 4,27 ($AB_q$, $J_{AB}$ = 8,6 Hz, $\delta_A$-$\delta_B$ = 45 Hz, 2H) ; 4,65 (s large, 1H) ; 4,75 ($AB_q$, $J_{AB}$ = 12 Hz, $\delta_A$-$\delta_B$ = 90 Hz, 2H) ; 4,77 (pseudo t, J = 5 Hz, 2H) ; 4,95 (d, J = 55,6 Hz, 2H) ; 4,98 (d, J = 8,3 Hz, 1H) ; 5,44 (s large, 1H) ; 5,58 (dd, J = 7 Hz et 10,6 Hz, 1H) ; 5,72 (d, J = 7 Hz, 1H) ; 6,26 (s, 1H) ; 6,31 (t déformé, J = 8,3 Hz et 9,2 Hz, 1H) ; 7,31-7,64 (m, 8H) ; 8,05-8,13 (m, 2H).

Dans un ballon de 10 cm3, muni d'une agitation magnétique, on introduit, sous atmosphère d'argon, 80 mg (0,0606 mmole) du produit obtenu ci-dessus en solution dans 3,2 cm3 de méthanol. On ajoute successivement 3,2 cm3 d'acide acétique glacial et 320 mg du couple zinc-cuivre (préparé à partir de 20 g de zinc en poudre et de 3 g de sulfate de cuivre hydraté). On chauffe à 60°C pendant 30 minutes. Après refroidissement, on ajoute 50 cm3 d'acétate d'éthyle. Le mélange est filtré sur célite sous pression réduite. Les solides sont lavés avec 5 fois 10 cm3 d'acétate d'éthyle. Les phases organiques réunies sont lavées 3 fois avec 5 cm3 d'eau et 2 fois avec 5 cm3 d'une solution aqueuse saturée de chlorure de sodium et enfin séchées sur sulfate de sodium anhydre. Après filtration et concentration à sec sous pression réduite, le résidu obtenu (50 mg) est purifié par chromatographie préparative sur couche mince de silice en éluant avec un mélange chlorure de méthylène-méthanol (94-6 en volumes ; 2 passages).

On obtient ainsi, avec un rendement de 78 %, 38 mg (0,0471 mmole) de t-butoxycarbonylamino-3 phényl-3 hydroxy-2 propionate-(2R,3S) d'acétoxy-4 benzyloxy-2α époxy-5β,20 trihydroxy-1,7β,10β oxo-9 taxène-11 yle-13α dont les caractéristiques, qui sont identiques à celles données dans la littérature, sont les suivantes:

- spectre infra-rouge (film) : 3450, 3100, 3050, 2950, 2920, 2890, 2850, 1730, 1710, 1600, 1580, 1490, 1450, 1390, 1370, 1315, 1270, 1245, 1160, 1105, 1095, 1070, 1020, 980, 910, 730 et 710 $cm^{-1}$
- spectre de résonance magnétique nucléaire du proton (chlorofore deutéré ; 300 MHz) : 1,13 (s, 3H) ; 1,24 (s, 3H) ; 1,34 (s, 9H) ; 1,76 (s, 3H) ; 1,85 (s, 3H) ; 1,74-1,85 (m, 1H) ; 2,26-2,29 (m, 2H) ; 2,37 (s, 3H) ; 254-2,66 (m, 1H) ; 3,32 (ps s, 1H); 3,92 (d, J = 7 Hz, 1H) ; 4,18-4,30 (m, 1H) ; 4,24 ($AB_q$, $J_{AB}$ = 8,25 Hz, $\delta_A$-$\delta_B$ = 36,5 Hz, 2H) ; 4,62 (ps s, 1H) ; 4,94 (d, J = 7,4 Hz, 1H) ; 5,20 (s, 1H) ; 5,25 (ps d, 1H) ; 5,42 (ps d, 1H) ; 5,68 (d, J = 7 Hz, 1H) ; 6,21 (t, J = 8,3 Hz, 1H) ; 7,31-7,40 (m, 5H) ; 7,43-7,52 (m, 2H) ; 7,59-7,64 (m, 1H) ; 8,09-8,12 (m, 2H).

## EXEMPLE 9

Dans un monocol de 15 cm3, muni d'un système d'agitation magnétique, on introduit sous atmosphère d'argon, 200 mg (0,67 mmole) de benzoylamino-3 hydroxy-2 phényl-3 propionate de méthyle-(2R,3S) et 2,4 cm3 d'acétronitrile. On ajoute ensuite, à la suspension résultante, 431 mg (2,01 mmoles) d'"éponge à protons", 1,42 cm3 (940 mg, 13,4 mmoles) de méthyl-2 butène-2 et 7 grains de tamis moléculaire 3 Å. On agite le mélange réactionnel résultant pendant 5 minutes à une température voisine de 20°C puis on ajoute 325 mg (1,34 mmole) de bromure de trichloro-2,2,2 éthoxyméthyle. On laisse réagir pendant 30 heures à une température voisine de 20°C. Le mélange réactionnel est fortement hétérogène. On ajoute encore successivement 431 mg (2,01 mmoles) d'"éponge à protons" et 585 mg (2,41 mmoles) de bromure de trichloro-2,2,2 éthoxyméthyle. Après 24 heures de réaction à une température voisine de 20°C, la réaction n'est pas terminée, on ajoute encore successivement 215,4 mg (1,05 mole) d'"éponge à protons" et 162,4 mg (0,67 mmole) de bromure de trichloro-2,2,2 éthoxyméthyle. On laisse réagir pendant encore 42 heures à une température voisine de 20°C. Après 96 heures de réaction au total, bien que la réaction ne soit pas encore terminée, on l'arrête. On traite alors le mélange réactionnel par 5 cm3 d'une solution aqueuse saturée de bicarbonate de sodium pendant 10 minutes. On ajoute ensuite 100 cm3 de chlorure de méthylène et 10 cm3 d'eau. Après décantation, la phase aqueuse est extraite 2 fois avec 15 cm3 de chlorure de méthylène. Les phases organiques réunies sont lavées avec 2 fois 5 cm3 d'une solution aqueuse d'acide chlorhydrique 2N, 4 fois avec 10 cm3 d'eau et 2 fois avec

10 cm3 d'une solution aqueuse saturée de chlorure de sodium. Après séchage sur sulfate de sodium anhydre, filtration et concentration à sec sous pression réduite, on obtient 950 mg de produit brut qui est purifié par chromatographie sur silice en éluant un mélange hexane-éther (85-15 en volumes). On obtient ainsi, avec un rendement de 72 %, 222 mg (0,482 mmole) de benzoylamino-3 phényl-3 (trichloro-2,2,2 éthoxyméthoxy)-2 propionate de méthyle-(2R,3S) dont les caractéristiques sont les suivantes :

- point de fusion: 70-71°C
- pouvoir rotatoire: $[\alpha]^{25}_D$ = +7,2° (c = 1,16; chloroforme)
- spectre infra-rouge (film) : principales bandes d'absorption caractéristiques à 3450, 3340, 3070, 3040, 2950, 2920, 1750, 1660, 1602, 1580, 1518, 1485, 1435, 1355, 1315, 1290, 1270, 1210, 1170, 1125, 1080, 1018, 805 $cm^{-1}$
- spectre de résonance magnétique nucléaire du proton (chloroforme deutéré ; 300 MHz) : 3,49 ($AB_q$, $J_{AB}$ = 11,3 Hz, $\delta_A$-$\delta_B$ = 126,5 Hz, 2H) ; 3,79 (s, 3H) ; 4,69 (d, J=1,8 Hz, 1H) ; 4,86 ($AB_q$, $J_{AB}$ = 7,1 Hz, $\delta_A$-$\delta_B$ = 32,2 Hz, 2H) ; 5,81 (dd, J=1,8 Hz et 9 Hz, 1H) ; 7,05 (d, J=9 Hz, 1H) ; 7,26-7,56 (m, 8H) ; 7,80-7,83 (m, 2H).
- spectre de résonance magnétique nucléaire du $^{13}C$ (75,47 Hz ; chloroforme deutéré) : 52,49 ($CH_3$) ; 56,76 (CH) ; 77,19 (CH) ; 79,39 ($CH_2$) ; 94,68 ($CH_2$) ; 96,09 (C) ; 126,43 (CH) ; 127,02 (CH) ; 127,89 (CH) ; 128,38 (CH) ; 128,58 (CH) ; 131,70 (CH) ; 134,00 (C) ; 138,34 (C) ; 166,75 (C) ; 169,88 (C)
- spectre de masse (i.c. ;$NH_3$ + isobutane) : ion molécuaire (massif) : $M^+$ (460,5) ; $M^+$ +2 (462,5) ; $M^+$ +4 (464,5) ; $M^+$ +6 (466,5) : pics dans le rapport 100-97,5-31,7 et 3,4.
- analyse élémentaire ($C_{20}H_{20}O_5NCl_3$)

| | | | | | | |
|---|---|---|---|---|---|---|
| calculé | C % | 52,14 | H % | 4,38 | N % | 3,04 |
| trouvé | | 52,11 | | 4,49 | | 3,04 |

EXEMPLE 10

Dans un ballon de 50 cm3, muni d'un système d'agitation magnétique, on introduit 107 mg (0,232 mmole) de l'ester obtenu à l'exemple 9 et 14 cm3 de méthanol. On ajoute ensuite, à la solution obtenue, 7 cm3 d'eau distillée et 98 mg (0,71 mmole) de carbonate de potassium. On agite pendant 20 heures à une température voisine de 20°C puis on évapore sous pression réduite le méthanol et l'eau. Le résidu obtenu est dissous dans 10 cm3 d'eau. La phase aqueuse basique résultante est lavée 3 fois avec 10 cm3 d'éther. La phase aqueuse est refroidie à 0°C puis est acidifiée par 2 cm3 d'acide chlorhydrique 2M (aqueux), sous forte agitation, en présence de 20 cm3 de chlorure de méthylène. Après décantation, elle est extraite 8 fois avec 20 cm3 de chlorure de méthylène. Les phase organiques réunies sont lavées 3 fois avec 10 cm3 d'eau puis 1 fois avec 10 cm3 d'une solution aqueuse saturée de chlorure de sodium. Après séchage sur sulfate de magnésium anhydre, filtration et concentration à sec sous pression réduite, on obtient, avec un rendement de 88 %, 91 mg (0,204 mmole) d'acide benzoylamino-3 phényl-3 (trichloro-2,2,2 éthoxyméthoxy)-2 propionique-(2R,3S) dont les caractéristiques sont les suivantes :

- pouvoir rotatoire: $[\alpha]^{25}_D$ = +22,8° (c = 1,02 ; chloroforme)
- spectre infra-rouge (film) : principales bandes d'absorption caractéristiques à 3440, 3700-2300, 3070, 3040, 2975, 2925, 1745, 1640, 1602, 1580, 1520, 1485, 1295, 1265, 1205, 1175, 1135, 1080, 1020, 810 $cm^{-1}$
- spectre de résonance magnétique nucléaire du proton (chloroforme deutéré ; 300 MHz): 2,80 (s large, 1H + $H_2O$) ; 3,50 ($AB_q$, $J_{AB}$ = 12 Hz, $\delta_A$-$\delta_B$ = 121,4 Hz, 2H) ; 4,73 (d, J=1,7 Hz, 1H) ; 4,88 ($AB_q$, $J_{AB}$ = 7,3 Hz, $\delta_A$-$\delta_B$ = 34,7 Hz, 2H) ; 5,93 (dd, J=1,7 Hz et 9 Hz, 1H) ; 7,19 (d, J-=9 Hz, 1H) ; 7,25-7,54 (m, 8H) ; 7,79-7,82 (m, 2H)
- spectre de résonance magnétique nucléaire du $^{13}C$ (75,47 Hz ; chloroforme deutéré) : 54,87 (CH) ; 76,84 (CH) ; 79,58 ($CH_2$); 94,86 ($CH_2$) ; 96,18 (C) ; 126,52 (CH) ; 127,33 (CH) ; 128,14 (CH) ; 128,73 (CH) ; 128,82 (CH) ; 132,12 (CH) ; 133,58 (C) ; 138,18 (C) ; 168,06 (C) ; 170,91 (C)
- spectre de masse (i.c.; $NH_3$ + isobutane) : ion moléculaire (massif) : $M^+$ (446,5) ; $M^+$ + 2 (448,5) ; $M^+$ +4 (450,5) ; $M^+$ +6 (452,5) : 4 pics dans le rapport 100-97,5-31,7-3,4
- analyse élémentaire ($C_{19}H_{18}O_5NCl_3$, $1H_2O$)

| | | | | | | |
|---|---|---|---|---|---|---|
| calculé | C % | 49,11 | H % | 4,34 | N % | 3,01 |
| trouvé | | 49,05 | | 4,33 | | 3,09 |

EXEMPLE 11

Dans un ballon monocol de 50 cm3 muni d'un système d'agitation magnétique, on introduit 267 mg (1 mmole) de (+)-phényl-1 hydroxy-2 butène-3 syn et 3,5 cm3 d'acétonitrile anhydre. A la suspension obtenue, on ajoute successivement 2,12 cm3 (1,4 g, 20 mmoles) de méthyl-2 butène-2, 14 grains de tamis moléculaire 3Å et 614 mg (2,86 mmoles) d'"éponges à protons". On agite le mélange réactionnel pendant 5 minutes à une température voisine de 20°C puis on ajoute 481 mg (1,98 mmole) de bromure de trichloro-2,2,2 éthoxyméthyle. On laisse réagir pendant 23 heures à une température voisine de 20°C puis on ajoute encore 643 mg (3 mmoles) d'"éponge à protons" et 485 mg (2 mmoles) de bromure de trichloro-2,2,2 éthoxyméthyle puis on agite pendant 24 heures. On additionne encore 242 mg (1 mmole) de bromure de trichloro-2,2,2 éthoxyméthyle et on laisse réagir pendant 20 heures. La réaction terminée, on ajoute 10 cm3 d'une solution aqueuse saturée d'hydrogénocarbonate de sodium et on agite pendant 15 minutes. On ajoute 100 cm3 de dichlorométhane. La phase aqueuse séparée est extraite 2 fois avec 10 cm3 de dichlorométhane. Les phases organiques réunies sont lavées 2 fois avec 10 cm3 d'une solution aqueuse d'acide chlorhydrique 2M, puis 4 fois avec 15 cm3 d'eau et 2 fois avec 15 cm3 d'une solution aqueuse saturée de chlorure de sodium. Après séchage sur sulfate de sodium anhydre, filtration et concentration à sec sous pression réduite, le résidu jaune obtenu est purifié par chromatographie sur colonne de gel de silice en éluant avec un mélange dichlorométhane-éther (98-2 en volumes). On obtient ainsi, avec un rendement de 63 %, 270 mg de (+)-phényl-1 benzoylamino-1 (trichloro-2,2,2 éthoxyméthoxy)-2 butène-3 syn dont les caractéristiques physiques sont les suivantes :

- point de fusion : 91-92°C
- pouvoir rotatoire: $[\alpha]^{25}_D = +31°$ (c = 0,8, chloroforme)

Les spectres infra-rouge et de résonnance magnétique nucléaire du proton du produit obtenu sont identiques à ceux du produit décrit dans l'exemple 14.

EXEMPLE 12

Dans un ballon monocol de 10 cm3 muni d'un système d'agitation magnétique, on introduit sous atmosphère d'argon 202 mg (0,47 mmole) de (+)-phényl-1 benzoylamino-1 (trichloro-2,2,2 éthoxyméthoxy)-2 butène3 syn et 1,2 cm3 d'acétonitrile anhydre. Au mélange obtenu, on ajoute successivement 1,2 cm3 de tétrachlorure de carbone, 1,8 cm3 d'eau et, sous forte agitation, 317 mg (3,77 mmoles) d'hydrogénocarbonate de sodium solide. On ajoute ensuite, par petites portions, 683 mg (3,19 mmoles) de periodate de sodium. On agite ensuite le mélange réactionnel fortement hétérogène pendant 5 minutes (dégagement gazeux) puis on ajoute, en une seule fois, 24,8 mg de $RuCl_3$. On laisse agir le mélange réactionnel devenu noir pendant 40 heures à une température voisine de 15°C. La réaction terminée, on additionne 15 cm3 d'eau. La phase aqueuse basique est lavée 3 fois par 20 cm3 d'éther. La phase aqueuse est refroidie à 0°C puis, sous forte agitation, est traitée, en présence, de 50 cm3 de dichlorométhane, par 2,5 cm3 d'une solution aqueuse d'acide chlorhydrique 2M. La phase aqueuse acide ainsi obtenue est extraite 7 fois avec 70 cm3 de dichlorométhane. Les phases organiques réunies sont lavées 4 fois avec 10 cm3 d'eau et 1 fois avec 10 cm3 d'une solution aqueuse saturée de chlorure de sodium. Après séchage de la phase organique sur sulfate de sodium anhydre, filtration et concentration à sec sous pression réduite, on obtient, avec un rendement de 76 %, 160 mg d'acide (+)-benzoylamino-3 phényl-3 (trichloro-2,2,2 éthoxyméthoxy)-2 propionique (2R,3S) sous forme de cristaux blancs dont les caractéristiques physiques et spectroscopiques sont identiques à celles du même acide obtenu dans l'exemple 10.

EXEMPLE 13

Dans un monocol de 250 cm3, muni d'un système d'agitation magnétique, on introduit, sous atmosphère d'argon, 5,4 cm3 de tétraméthylpéridine (32 mmoles) et 6,5 cm3 de tétrahydrofuranne anhydre. On refroidit la solution obtenue à -60°C puis on ajoute, goutte à goutte, 20 cm3 d'une solution de n.butyllithium 1,6M dans l'hexane. On laisse la température de la solution hétérogène remonter à -10°C en 1 heure. La solution de tétraméthylpipéridure de lithium est refroidie à -60°C puis on ajoute, en 20 minutes, une solution de 2,16 g de N-benzoylbenzamide (10,25 mmoles) et de 3,4 cm3 de N,N,N',N'-tétraméthyléthylènediamine (22,5 mmoles) dans 8,5 cm3 de tétrahydrofuranne. Après 1 heure d'agitation à -60°C, on ajoute, en une seule fois, 2,4 cm3 d'acroléine (36 mmoles) fraîchement distillée. On laisse réagir pendant 15 minutes à -60°C puis on hydrolyse par addition de 20 cm3 d'eau. Le mélange réactionnel est extrait 3 fois avec 30 cm3 de dichlorométhane. Les phases organiques réunies sont lavées avec 10 cm3 d'une solution d'acide chlorhydrique 1N, 2 fois avec 20 cm3 d'eau puis 1 fois avec 10 cm3 d'une solution aqueuse saturée de chlorure de sodium, puis séchées sur sulfate de sodium anhydre. Après filtration et concentration sous pression réduite, on obtient un résidu (4,3 g)

qui est purifié par chromatographie sur gel de silice en éluant avec un mélange dichlorométhane-acétate d'éthyle (9-1 en volumes). On obtient ainsi, avec un rendement de 23 %, 630 mg de (±)-phényl-1 benzoylamino-1 hydroxy-2 butène-3 syn et, avec un rendement de 15 %, 409 mg de (±)-phényl-1 benzoylamino-1 hydroxy-2 butène-3 anti.

Les caractéristiques du (±)-phényl-1 benzylamino-1 hydroxy-2 butène-3 syn sont les suivantes:
- point de fusion : 137- 139°C (dichlorométhane-cyclohexane)
- spectre infra-rouge (film) : principales bandes d'absorption caractéristiques à 3300, 1620, 1525, 1510, 1335, 1295, 1120, 1080, 995, 920 et 700 $cm^{-1}$
- spectre de résonance magnétique nucléaire du proton (chloroforme deutéré ; 200 MHz) : 2,40 (d, J = 3,9 Hz, 1H) ; 4,55 (dd, J = 3,5 Hz, 3,5 Hz et 5 Hz, 1H) ; 5,23 (dt, J = 1,5 Hz et 10,5 Hz, 1H) ; 5,26 (dd, J = 3,5 Hz et 7,6 Hz, 1H) ; 5,40 (dt, J = 1,5 Hz et 17,1 Hz, 1H) ; 5,94 (ddd, J = 5 Hz, 10,5 Hz et 17,1 Hz, 1H) ; 6,98 (d, J = 7,6 Hz, 1H) ; 7,24-7,54 (m, 8H) ; 7,80-7,83 (m, 2H)
- spectre de résonnance magnétique nucléaire du $^{13}C$ (chloroforme deutéré ; 75,47 MHz) : 57,71 (CH) ; 75,31 (CH) ; 116,58 ($CH_2$) ; 126,89 (CH) ; 127,04 (CH) ; 127,68 (CH) ; 128,56 (CH) ; 128,72 (CH) ; 131,60 (CH) ; 134,31 (C) ; 137,41 (CH) ; 139,60 (C) ; 167,54 (C).

Les caractéristiques du (±)-phényl-1 benzylamino-1 hydroxy-2 butène-3 anti sont les suivantes :
- spectre infra-rouge (nujol): principales bandes d'absorption caractéristiques à 3300, 1620, 1600, 1580, 1520, 1300, 1115, 1080, 1060, 1035, 985, 920, 870, 820, 800, 750 et 700 $cm^{-1}$
- spectre de résonnance magnétique nucléaire du proton (chloroforme deutéré ; 200 MHz) : 2,43 (s large, 1H) ; 4,60 (m, 1H) ; 5,23 (dt, J = 1,5 Hz et 10,4 Hz, 1H) ; 5,33 (dd, J = 4 Hz et 8 Hz, 1H) ; 5,34 (dt, J = 1,5 Hz et 17 Hz, 1H) ; 5,79 (ddd, J = 5 Hz, 10,4 Hz et 17 Hz, 1H) ; 6,88 (d, J = 8 Hz, 1H) ; 7,3-7,54 (m, 8H) ; 7,78-7,82 (m, 2H)
- spectre de résonnance magnétique nucléaire du $^{13}C$ (chloroforme deutéré; 75,47 MHz) : 58,23 (CH) ; 75,28 (CH) ; 117,29 ($CH_2$) ; 126,94 (CH) ; 127,52 (CH) ; 127,80 (CH) ; 128,53 (CH) ; 128,58 (CH) ; 131,64 (CH) ; 134,14 (C) ; 136,30 (CH) ; 137,56 (C) ; 167,23 (C).

## EXEMPLE 14

Dans un ballon monocol de 25 cm3, muni d'un système d'agitation magnétique, on introduit, sous atmosphère d'argon, 100 mg de (±)-phényl-1 benzoylamino-1 hydroxy-2 butène-3 syn (0,37 mmole) et 1,3 cm3 d'acétonitrile anhydre. A la suspension obtenue, on ajoute successivement 0,8 cm3 de méthyl-2 butène-2 (530 mg ; 7,55 mmoles), 4 grains de tamis moléculaire 3Å et 230 mg d'"éponge à protons" (1,07 mmole). On agite le mélange réactionnel pendant 5 minutes à une température voisine de 20°C puis on ajoute 180 mg de bromure de trichloro-2,2,2 éthoxyméthyle (0,74 mmole). On laisse agir pendant 21 heures à une température voisine de 20°C puis on ajoute 115 mg d'"éponge à protons" (0,54 mmole) et 90 mg de bromure de trichloro-2,2,2 éthoxyméthyle (0,37 mmole) puis on agite pendant 19 heures. On ajoute 10 cm3 d'une solution aqueuse saturée d'hydrogénocarbonate de sodium et agite pendant 15 minutes.

On ajoute 40 cm3 de dichlorométhane. La phase aqueuse séparée est extraite 2 fois avec 10 cm3 de dichlorométhane. Les phases organiques réunies sont lavées 2 fois avec 5 cm3 d'une solution aqueuse d'acide chlorhydrique 2M puis 4 fois avec 5 cm3 d'eau et enfin 2 fois avec 5 cm3 d'une solution saturée de chlorure de sodium. Après séchage sur sulfate de sodium anhydre, filtration et concentration sous pression réduite, le résidu obtenu (306 mg) est purifié par chromatographie sur colonne de gel de silice en éluant avec un mélange dicchlorométhane-acétate d'éthyle (98-2 en volumes). On obtient ainsi 144 mg d'un produit solide qui est recristallisé dans un mélange éther-hexane. On obtient ainsi, avec un rendement de 79 %, 125 mg de (±)-phényl-1 benzoylamino-1 (trichloro-2,2,2 éthoxyméthoxy)-2 butène-3 syn dont les caractéristiques sont les suivantes :
- point de fusion: 94-95°C (éther-hexane)
- spectre infra-rouge (film) : principales bandes d'absorption caractéristiques à 3425, 3300, 3050, 3020, 2900, 1630, 1600, 1570, 1510, 1480, 1290, 1160, 1110, 1070, 1010, 930, 800 et 710 $cm^{-1}$
- spectre de résonnance magnétique nucléaire du proton (chloroforme deutéré; 200 MHz) : 3,43 ($AB_q$, $J_{AB}$ = 11,8 Hz ; $\delta_A$-$\delta_B$ = 80,1 Hz, 2H) ; 4,56 (dd, J = 2,5 Hz et 8 Hz, 1H) ; 4,79 (ABq, $J_{AB}$ = 7,0 Hz ; $\delta_A$-$\delta_B$ = 17,2 Hz, 2H) ; 5,41 (dd, J = 2,5 Hz et 8,8 Hz, 1H) ; 5,42 (d, J = 17,2 Hz, 1H) ; 5,43 (d, J = 10,2 Hz, 1H) ; 5,88 (ddd, J = 6,8 Hz, 10,2 Hz et 17,2 Hz, 1H) ; 6,99 (d, J = 8,8 Hz, 1H) ; 7,26-7,54 (m, 8H) ; 7,82-7,87 (m, 2H)
- spectre de résonnance magnétique du $^{13}C$ (chloroforme deutéré; 50,3 MHz ): 56,24 (CH) ; 79,19 ($CH_2$) ; 79,71 (CH) ; 92,86 ($CH_2$) ; 96,89 (C) ; 119,76 ($CH_2$) ; 126,66 (CH) ; 127,00 (CH) ; 127,65 (CH) ; 128,57 (CH) ; 128,65 (CH) ; 131,67 (CH) ; 134,15 (CH) ; 134,23 (C) ; 139,66 (C) ; 166,87 (C)
- spectre de masse (i.c. ; $NH_3$ + isobutane) : ion moléculaire (massif) : $M^+$ (428,5) ; $M^+$ +2 (430,5) ; $M^+$ +4 (432,5) ; $M^+$ +6 (434,5) ; 4 pics dans le rapport 100-97,5-31,7 et 3,4
- analyse élémentaire ($C_{20}H_{20}O_3NCl_3$)

| | | | | | | |
|---|---|---|---|---|---|---|
| calculé | C % | 56,03 | H % | 4,70 | N % | 3,27 |
| trouvé | | 55,88 | | 4,72 | | 3,24 |

EXEMPLE 15

Dans un ballon monocol de 5 cm3, muni d'un système d'agitation magnétique, on introduit, sous atmosphère d'argon, 52 mg de (±)-phényl-1 benzoylamino-1 (trichloro-2,2,2 éthoxyméthoxy)-2 butène-3 syn obtenu à l'exemple 12 (0,12 mmole) en solution dans 0,25 cm3 d'acétonitrile. On ajoute ensuite sucessivement 0,25 cm3 de tétrachlorure de carbone, 0,37 cm3 d'eau distillée et, sous agitation, 65,5 mg d'hydrogénocarbonate de sodium solide (0,78 mmole). On ajoute ensuite, par petites portions, 141 mg de periodate de sodium (0,66 mmole). On agite pendant 5 minutes puis on ajoute, en une seule fois, 5,2 mg de $RuCl_3$. On laisse réagir le mélange réactionnel devenu noir pendant 72 heures à une température de 16-17°C. On ajoute à nouveau 3 mg de catalyseur puis on agite pendant 44 heures 25-30°C. On ajoute alors 3 cm3 d'eau. La phase aqueuse basique est lavée 3 fois avec 4 cm3 d'éther. La phase aqueuse est refroidie à 0°C puis, sous forte agitation, est traitée, en présence de 10 cm3 de dichlorométhane, par 0,5 cm3 d'une solution aqueuse d'acide chlorhydrique 2M. La phase aqueuse acide ainsi obtenue est extraîte 8 fois avec 10 cm3 de dichlorométhane. Les phases organiques réunies sont lavées 4 fois avec 2 cm3 d'eau et 1 fois avec 2 cm3 d'une solution saturée de chlorure de sodium. Après séchage de la phase organique sur sulfate de sodium anhydre, filtration et concentration à sec, on obtient 43 mg d'un solide blanc qui est recristallisé dans un mélange éther-hexane. On obtient ainsi, avec un rendement de 71 %, 38 mg d'acide (±)-benzoylamino-3 phényl-3 (trichloro-2,2,2 éthoxyméthoxy)-2 propionique syn dont les caractéristiques sont les suivantes :

- point de fusion: 132-133°C (éther-hexane)
- spectre infra-rouge (film) : bandes d'absorption caractéristiques à 3700-2300, 3440, 3300, 3070, 3040, 2975, 2925, 1745, 1640, 1602, 1580, 1520, 1485, 1295, 1265, 1205, 1175, 1135, 1080, 1020, 810, 720 et 700 $cm^{-1}$
- spectre de résonance magnétique nucléaire du proton (chloroforme deutéré ; 300 MHz) : 2,80 [s large, H (+$H_2O$)] ; 3,50 (ABq, $J_{AB}$ = 12 Hz ; $\delta_A$-$\delta_B$ = 121,4 Hz, 2H) ; 4,73 (d, J = 1,7 Hz, 1H) ; 4,88 ($AB_q$, $J_{AB}$ = 7,3 Hz ;, $\delta_A$-$\delta_B$ = 34,7 Hz, 2H) ; 5,93 (dd, J = 1,7 Hz et 9 Hz, 1H) ; 7,19 (d, J = 9 Hz, 1H) ; 7,25-7,54 (m, 8H) ; 7,79-7,82 (m, 2H)
- spectre de résonance magnétique nucléaire du $^{13}C$ (chloroforme deutéré ; 75,47 MHz) : 54,87 (CH) ; 76,84 (CH) ; 79,58 ($CH_2$) ; 94,86 ($CH_2$) ; 96,18 (C) ; 126,52 (CH) ; 127,33 (CH) ; 128,14 (CH) ; 128,73 (CH) ; 128,82 (CH) ; 132,12 (CH) ;133,58 (C) ; 138,18 (C) ; 168,06 (C) ; 170,91 (C)
- spectre de masse (i.c. ; $NH_3$ + isobutane) : ion moléculaire (massif) : $M^+$ (446,5) ; $M^+$ +2 (448,5) ; $M^+$ +4 (450,5) ; $M^+$ +6 (452,5) : 4 pics dans le rapport 100-97,5-31,7-3,4
- analyse élémentaire ($C_{19}H_{18}O_5NCl_3$, $1H_2O$)

| | | | | | | |
|---|---|---|---|---|---|---|
| calculé | C % | 49,11 | H % | 4,34 | N % | 3,01 |
| trouvé | | 49,05 | | 4,33 | | 3,09 |

EXEMPLE 16

Dans un proton monocol de 10 cm3, on introduit 115 mg d'acide (±)-benzoylamino-3 phényl-3 (trichloro-2,2,2 éthoxyméthoxy)-2 propionique (0,26 mmole) en solution dans 0,5 cm3 d'acétone. On ajoute ensuite 43 mg de (-)-pseudoéphédrine (0,26 mmole) puis on chauffe le mélange réactionnel jusqu'à l'obtention d'une solution homogène. On refroidit alors à -10°C pendant 19 heures. Les cristaux blancs obtenus sont isolés et séchés. On obtient ainsi 80 mg de sel d'éphédrine que l'on remet en solution dans 0,5 cm3 d'acétone. On refroidit pendant 15 heures à -10°C. On obtient ainsi, après séparation par filtration, 30 mg de sel sous forme de cristaux blancs. Après hydrolyse en milieu acide (acide chlorhydrique aqueux 1N ; dichlorométhane ; 20°C ; extraction habituelle), on obtient avec un rendement de 19 %, 22 mg d'acide (+)-bezoylamino-3 phényl-3 (trichloro-2,2,2 éthoxyméthoxy)-2 propionique (2R,3S) dans lequel le rapport énantiomérique (déterminé par le spectre de résonance magnétique nucléaire du proton et du $^{19}F$ des esters de Mosher des alcools-esters méthyliques racémique et optiquement actif correspondants) est 93-7.

Les eaux-mères issues de la seconde cristallisation, après concentration, fournissent 50 mg de résidu. Le résidu est mis en solution dans 0,5 cm3 d'acétone puis on refroidit pendant 24 heures à -10°C. On obtient ainsi, après filtration, 13 mg de sel sous forme de cristaux blancs que l'on hydrolyse dans les conditions décrites ci-dessus. On obtient ainsi, avec un rendement de 7 %, 8 mg d'acide (+)-benzoylamino-3 phényl-3 (trichloro-2,2,2 éthoxyméthoxy)-2 propionique (2R,3S) identique à celui obtenu ci-dessus.

Le rendement de la résolution est de 52 %.

EXEMPLE 17

Dans un ballon monocol de 5 cm3, muni d'un poème d'agitation magnétique, on introduit, sous atmosphère d'argon, 62 mg d'acide (+)-benzoylamino-3 phényl-3 (trichloro-2,2,2 éthoxyméthoxy)-2 propionique (2R,3S) (0,139 mmole) en solution dans 1,16 cm3 de toluène anhydre. On ajoute ensuite 30 mg de dipyridyl-2 carbonate (0, 139 mmole) et laisse réagir pendant 5 minutes à une température voisine de 20°C. On ajoute alors, en une seule fois, un mélange de 16,1 mg de triéthylsilyl-7 baccatine III (0,023 mmole) et de 5,6 mg de N,N-diméthylamino-4 pyridine (0,046 mmole). On laisse réagir pendant 3-4 minutes à une température voisine de 20°C puis on chauffe à 70-72°C pendant 48 heures. La réaction terminée, on dilue le mélange réactionnel résultant par 60 cm3 d'acétate d'éthyle. La phase organique est lavée par 3 cm3 d'eau, 2 fois avec 3 cm3 d'une solution aqueuse saturée d'hydrogénocarbonate de sodium, 4 fois avec 3 cm3 d'eau et 2 fois avec 3 cm3 d'une solution saturée de chlorure de sodium puis elle est séchée sur sulfate de sodium anhydre. Après filtration et évaporation du solvant sous pression réduite, le résidu obtenu (75 mg) est purifié par chromatographie préparative sur couche mince de silice en éluant avec un mélange éther-dichlorométhane (8-92 en volumes) (3 passages).

On obtient ainsi, avec un rendement de 94 %, 24,4 mg de (+)-benzoylamino-3 phényl-3 (trichloro-2,2,2 éthoxyméthoxy)-2 propionate de diacétoxy-4,10 benzoyloxy-2α époxy-5β,20 hydroxy-1 oxo-9 triéthylsilyloxy-7β taxène-11 yle-13α, contenant, d'après le spectre de résonance magnétique nucléaire du proton à 300 Mz, 10 % d'épimère en C2′, dont les caractéristiques sont les suivantes :

- point de fusion: 137-145°C
- spectre infra-rouge (film) : principales bandes d'absorption caractéristiques à 3400, 2960, 2870, 1740, 1650, 1600, 1580, 1508, 1480, 1445, 1365, 1265, 1235, 1140, 1105, 1095, 1080, 1015, 985, 945, 818 et 710 $cm^{-1}$
- spectre de résonance magnétique nucléaire du proton (300 MHz ; chloroforme deutéré) ; 0,54-0,62 (m, 6H) ; 0,93 (t, J = 8 Hz, 9H) ; 1,19 (s, 3H) ; 1,23 (s, 3H) ; 1,70 (s, 3H) ; 1,85-1,99 (m, 1H) ; 2,03 (s, 3H) ; 2,10-2,20 (m, 1H) ; 2,17 (s, 3H) ; 2,33-2,60 (m, 2H) ; 2,52 (s, 3H) ; 3,60 (ABq, $J_{AB}$ = 11,7 Hz ; $\delta_A$-$\delta_B$ = 101,4 Hz, 2H) ; 3,82 (d, J = 7 Hz, 1H) ; 4,26 (ABq, $J_{AB}$ = 8,3 Hz ; $\delta_A$-$\delta_B$ = 35,5 Hz, 2H) ; 4,48 (dd, J = 6,7 Hz et 10,3 Hz, 1H) ; 4,81 (d, J = 2 Hz, 1H) ; 4,90 (ABq, $J_{AB}$ = 7,5 Hz ; $\delta_A$-$\delta_B$ = 17 Hz, 2H) ; 4,94 (d, J = 9,5 Hz, 1H) ; 5,70 (d, J = 7 Hz, 1H) ; 5,89 (dd, J =2 Hz et 9 Hz, 1H) ; 6,28 (t, J = 9 Hz, 1H) ; 6,44 (s, 1H) ; 7,01 (d, J = 9 Hz, 1H) ; 7,34-7,63 (m, 11H); 7,75-7,78 (m, 2H) ; 8,12-8,16 (m, 2H).

Dans un ballon monocol de 10 cm3, muni d'un système d'agitation magnétique, on introduit, sous atmosphère d'argon, 24,8 mg de l'ester obtenu ci-dessus en solution dans 0,9 cm3 de méthanol puis on ajoute successivement 0,9 cm3 d'acide acétique glacial et 60 mg de couple zinc-cuivre (préparé à partir de 20 g de zinc en poudre et de 3 g de $CuSO_4$, $H_2O$). On chauffe le mélange réactionnel résultant à 55-56°C pendant 1,5 heure. La réaction terminée, le mélange réactionnel est refroidi à une température voisine de 20°C puis filtré. Le solide est lavé avec 3 fois 0,6 cm3 d'éthanol à 95 %. A la solution lipide résultante on ajoute 70 mg d'acide chlorhydrique à 33 % (p/v) puis on laisse réagir pendant 8 heures à une température voisine de 20°C. La réaction terminée, on dilue le mélange réactionnel par 60 cm3 d'acétate d'éthyle. La phase organique est lavée 2 fois par 5 cm3 d'une solution aqueuse saturée en hydrogénocarbonate de sodium puis 4 fois avec 5 cm3 d'eau et 1 fois avec 5 cm3 d'une solution aqueuse saturée de chlorure de sodium. La phase organique est ensuite séchée sur sulfate de sodium anhydre. Après filtration et concentration sous pression réduite, le résidu obtenu (23 mg) est purifié par chromatographie préparative sur couche mince de silice en éluant avec un mélange méthanol-dichlorométhane (5-95 en volumes) (2 passages).

On obtient ainsi, avec un rendement de 73 %, 13,8 mg de taxol contaminé par 10 % de son épimère C2′.

Les spectres infra-rouge et de résonnance magnétique nucléaire du proton et du $^{13}C$ du taxol obtenu sont identiques à ceux du taxol naturel décrits dans la littérature.

## Revendications

**1 -** Procédé de préparation de nouveaux dérivés de la β-phénylisosérine de formule générale :

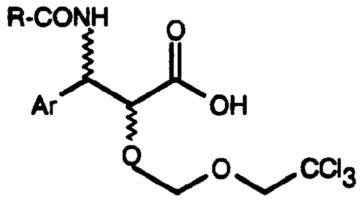

(I)

éventuellement sous forme de sel ou d'ester, dans laquelle R représente un radical t-butoxy ou phényle et Ar représente un radical aryle choisi parmi les radicaux phényle et α- ou β-naphtyle éventuellement substitués par un ou plusieurs atomes ou radicaux, identiques ou différents, choisis parmi les atomes d'halogène et les radicaux alcoyles, alcényles, alcynyles, alcoyloxy, alcoylthio, hydroxy, mercapto, amino, monoalcoylamino, dialcoylamino, acylamino, alcoxycarbonylamino, carboxy, carbamoyle, N,N-dialcoylcarbamoyle, cyano, nitro ou trifluorométhyle, caractérisé en ce que l'on fait agir un halogénure de trichloro-2,2,2 éthoxyméthyle sur un produit de formule générale:

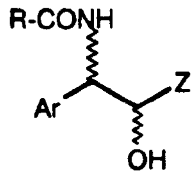

(V)

dans laquelle Z représente une fonction ester ($-COOR_2$) ou une double liaison vinylique ($-CH=CH_2$), puis éventuellement selon le cas, saponifie ou oxyde le produit ainsi obtenu, et isole le dérivé de la β-phénylisosérine qui est éventuellement dédoublé en ses énantiomères.

**2 -** Procédé selon la revendication 1 caractérisé en ce que l'action de l'halogénure de trichloro-2,2,2 éthoxyméthyle est réalisée dans un solvant organique tel que l'acétonitrile en présence d'un accepteur de proton à une température comprise entre 0 et 50°C.

**3 -** Procédé selon la revendication 2 caractérisé en ce que l'accepteur de proton est le 1,8-bis(diméthylamino) naphtalène.

**4 -** Procédé selon la revendication 2 caractérisé en ce que l'on opère en outre en présence de méthyl-2 butène-2.

**5 -** Procédé selon la revendication 1 caractérisé en ce que lorsque Z représente une fonction ester ($-COOR_2$), la saponification est effectuée au moyen d'une base minérale en milieu hydroalcoolique.

**6 -** Procédé selon la revendication 1 caractérisé en ce que lorsque Z représente une double liaison vinylique ($-CH=CH_2$), l'oxydation est effectuée au moyen de periodate de sodium en présence d'une quantité catalytique d'un dérivé du ruthénium ou au moyen de permanganate de potassium.

**7 -** Procédé selon la revendication 1 caractérisé en ce que le dérivé de la β-phénylisosérine est dédoublé en ses énantiomères par formation d'un sel avec une base optiquement active suivie de l'hydrolyse du sel ainsi obtenu.

**8 -** Nouveaux dérivés de la β-phénylisosérine de formule générale :

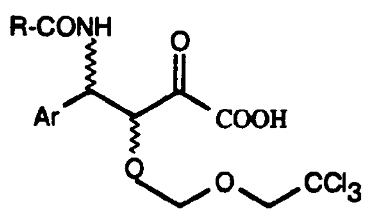

dans laquelle R et Ar sont définis comme dans la revendication 1, sous forme récémique ou énantiomériquement pure ou sous forme de leurs mélanges ainsi que leurs sels et leurs esters.

**9 -** Utilisation d'un produit obtenu selon le procédé de la revendication 1 pour la préparation de dérivés du taxane biologiquement actifs de formule générale :

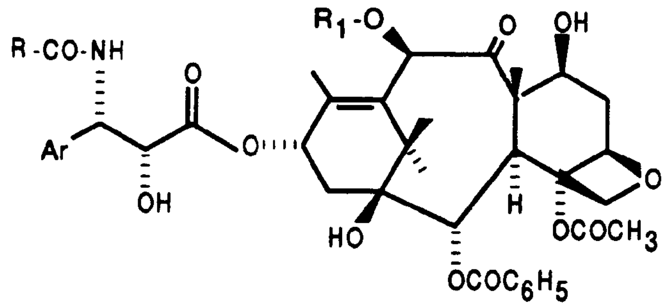

dans laquelle R et Ar sont définis comme dans la revendication 1 et $R_1$ représente un atome d'hydrogène ou un radical acétyle, caractérisé en ce que l'on estérifie un dérivé du taxane de formule générale:

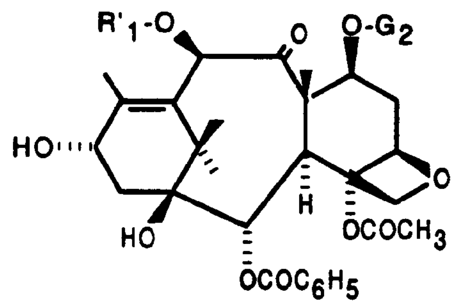

dans laquelle $R'_1$ représente un radical acétyle ou un groupement potecteur de la fonction hydroxy et $G_2$ représente un groupement protecteur de la fonction hydroxy tel que le groupement trichloro-2,2,2 éthoxycarbonyle ou trialcoylsilyle dont chaque partie alcoyle contient 1 à 4 atomes de carbone, au moyen d'un produit obtenu selon le procédé de la revendication 1, puis remplace les groupements protecteurs représentés par $R'_1$ et $G_2$ et le groupement trichloro-2,2,2 éthoxyméthyle par un atome d'hydrogène et, si nécessaire, effectue la résolution du produit obtenu pour isoler l'énantiomère 2R,3S.

**10 -** Utilisation selon la revendication 9 caractérisé en ce que l'estérification est effectuée en présence d'un agent de condensation tel que le N,N-dicyclohexylcarbodiimide ou le 2-dipyridylcarbonate et d'un agent d'activation tel que la N,N-diméthylamino-4 pyridine à une température comprise envi 20 et 80°C.

**11 -** Utilisation selon la revendication 9 caractérisé en ce que le remplacement des groupements protecteurs est effectué au moyen de zinc ou d'un couple zinc-cuivre dans l'acide acétique éventuellement en présence d'un alcool contenant 1 à 3 atomes de carbone ou, dans le cas du groupement protecteur trialcoylsilyle, au moyen d'HCl hydroalcoolique.

Office européen des brevets

# RAPPORT DE RECHERCHE EUROPEENNE

Numero de la demande
EP 92 40 2293

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A,D | EP-A-0 336 840 (C.N.R.S.)<br>* le document en entier * | 1-11 | C07C269/06<br>C07C231/12<br>C07D305/14<br>C07C233/83<br>C07C271/22 |
| A,D | EP-A-0 336 841 (RHONE-POULENC SANTE)<br>* le document en entier * | 1-11 | |
| P,X | WO-A-9 117 977 (RHONE POULENC RORER S.A.)<br>* page 1 - page 3; revendications * | 1-7,9-11 | |
| P,X | WO-A-9 117 976 (RHONE POULENC RORER S.A.)<br>* page 1 - page 3; revendications * | 1-7,9-11 | |

| DOMAINES TECHNIQUES RECHERCHES (Int. Cl.5 ) |
|---|
| C07C<br>C07D |

Le présent rapport a été établi pour toutes les revendications

| Lieu de la recherche | Date d'achèvement de la recherche | Examinateur |
|---|---|---|
| LA HAYE | 12 NOVEMBRE 1992 | SANCHEZ GARCIA J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)